# EUROPÄISCHE PATENTSCHRIFT

(11) **EP 3 928 090 B1**
(45) Veröffentlichungstag und Bekanntmachung des Hinweises auf die Patenterteilung: **23.11.2022**
(21) Anmeldenummer: 20705918.9
(22) Anmeldetag: 13.02.2020
(51) Int. Cl.: G01N 27/66, G01N 27/70, H03F 1/08, H03F 3/45, G01R 19/00

(54) **GASDETEKTOR MIT EINER IONISIERVORRICHTUNG**
GAS DETECTOR WITH AN IONISING DEVICE
DÉTECTEUR DE GAZ COMPRENANT UN DISPOSITIF IONISANT

(30) Priorität: 19.02.2019 DE 102019202242
(43) Veröffentlichungstag der Anmeldung: 29.12.2021
(73) Patentinhaber: Inficon GmbH, 50968 Köln (DE)
(72) Erfinder: ROLFF, Norbert, 50169 Horrem (DE)
(74) Vertreter: dompatent von Kreisler Selting Werner - Partnerschaft von Patent- und Rechtsanwälten mbB
(86) Internationale Anmeldenummer: PCT/EP2020/053756
(87) Internationale Veröffentlichungsnummer: WO 2020/169446

(56) Entgegenhaltungen:
- EP-A1- 0 219 557
- GB-A- 2 424 330
- US-A1- 2006 244 526
- US-A1- 2015 357 981

## Beschreibung

Die Erfindung betrifft einen Gasdetektor mit einer Ionisiervorrichtung zur Erzeugung von Ionen in Abhängigkeit von einem zu detektierenden Gastyp und zur Erzeugung eines elektrischen Potentials in Abhängigkeit von den Ionen.

Bei der Ionisiervorrichtung kann es sich um ein Massenspektrometer, wie zum Beispiel ein Sektorfeld-Massenspektrometer oder ein Quadrupol-Massenspektrometer zur Partialdruckmessung oder um ein Ionisations-vakuummeter zur Totaldruckmessung handeln. Derartige Ionisiervorrichtungen sind dazu ausgebildet, Ionen in Abhängigkeit von bestimmten Gastypen zu erzeugen. Mit einem Fänger wird der durch die Ionen erzeugte Ionenstrom aufgenommen.

Um den erzeugten Ionenstrom zu messen, wird eine Messvorrichtung eingesetzt, die aus dem Ionenstrom ein messbares Messpotential erzeugt. Die Messvorrichtung weist hierzu einen Stromverstärker, beispielsweise mit einem Operationsverstärker, auf, um den Ionenstrom zu verstärken. Über einen elektrischen Messwiderstand, beispielsweise in der Rückkopplung des Operationsverstärkers, wird aus dem verstärkten Strom ein messbares elektrisches Messpotential erzeugt.

Bei schnellen Vakuumanwendungen sind die bisherigen Stromverstärker im häufig verwendeten unteren Strombereich das langsamste Glied in der Signalkette. Dieser Nachteil soll erfindungsgemäß vermieden werden.

Ferner sind Anwendungen bekannt, bei denen der Ionenstrom mit einem Elektronenvervielfacher verstärkt werden. Dabei kann die Ansprechzeit verringert werden, allerdings mit geringerer Stabilität.

Insbesondere ist es herkömmlicherweise erforderlich, bei kleinen Strömen hochohmige Widerstände einzusetzen, die eine nennenswerte Kapazität zur Umgebung aufweisen. Wenn die Umgebung des Messwiderstands auf Massepotential gelegt wird, ergibt sich eine Überhöhung bei einem Signalsprung. Wenn hingegen ein Teil der Umgebung des Messwiderstands auf dem Ausgangssignal des Stromverstärkers liegt, steigt das Ausgangssignal langsam an.

Die herkömmlicherweise verwendeten Messwiderstände zur Messung des aus den Ionen erzeugten Stromes sind hochohmig im Bereich von circa 500 Gigaohm, um den Rauschanteil im Messsignal gering zu halten. Ein hochohmiger Messwiderstand erzeugt unerwünschte parasitäre Streukapazitäten.

US 2015/0357981 A1 beschreibt einen Transimpedanzverstärker mit einer Widerstandsanordnung zwischen dem Ausgang des Verstärkers und einem Eingang des Verstärkers.

US 2006/0244526 A1 beschreibt einen Transimpedanzverstärker mit einem Rückkopplungspfad.

GB 2,424,330 A beschreibt einen Transimpedanzverstärker mit einem abgeschirmten Rückkopplungswiderstand.

EP 0219557 A1 beschreibt eine Vorrichtung zur Überprüfung des Messsignalpfades einer Messeinrichtung.

Der Erfindung liegt die Aufgabe zugrunde, einen verbesserten Gasdetektor mit Ionisiervorrichtung zu schaffen.

Der erfindungsgemäße Gasdetektor wird definiert durch die Merkmale von Patentanspruch 1.

Der erfindungsgemäße Gasdetektor weist eine Ionisiervorrichtung der zuvor beschriebenen Art auf. Erfindungsgemäß ist der Messwiderstand zumindest teilweise von einem elektrischen Schirmwiderstand umgeben und in Längsrichtung des Messwiderstands an einander gegenüberliegenden Bereichen des Mess- und des Schirmwiderstands bis auf eine Abweichung von maximal 25 % dieselben Potenziale aufweisen. An dem Schirmwiderstand herrscht dadurch ein vergleichbarer oder nahezu gleicher Spannungsabfall wie an dem Messwiderstand. Die parasitären Streukapazitäten sind dadurch stromlos.

Sowohl der Messwiderstand als auch der elektrische Schirmwiderstand weisen einander gegenüberliegende äußere Enden auf, die jeweils zur elektrischen Kontaktierung der Widerstände dienen. An den beiden Enden des Messwiderstands sollen annähernd dieselben Potenziale anliegen wie an den entsprechenden Enden des Schirmwiderstands. Mit anderen Worten bedeutet das, dass an dem ersten Ende des Messwiderstands ungefähr, das heißt bis auf eine Abweichung von höchstens 25 %, dasselbe Potenzial anliegt wie an dem ersten Ende des Schirmwiderstands. An dem zweiten Ende des Messwiderstands liegt ebenfalls bis auf eine Abweichung von höchstens 25 % dasselbe Potenzial an wie an dem zweiten Ende des Schirmwiderstands. Im Ergebnis soll über den Messwiderstand und über den Schirmwiderstand räumlich verteilt dasselbe Potenzial abfallen.

Hierbei ist vorzugsweise das eine Ende des Messwiderstands mit dem einen Ende des Schirmwiderstands elektrisch verbunden, sodass die beiden Enden auf demselben Potenzial liegen. Die jeweils anderen Enden des Messwiderstands und des Schirmwiderstands können dann bis auf die Abweichung von höchstens 25 % auf Massenpotenzial liegen. Die Abweichung von höchstens 25 % kann beispielsweise daraus resultieren, dass das jeweilige Ende des Messwiderstands über die Eingangsseite eines Operationsverstärkers mit Masse verbunden ist, sodass zwischen dem betroffenen Anschluss des Messwiderstands und dem Massepotenzial noch die der Eingangsspannung des Operationsverstärkers entsprechende Potenzialdifferenz abfällt.

Der Widerstandswert des Schirmwiderstands sollte geringer sein als der Widerstandswert des Messwiderstands. Beispielsweise kann der Widerstandswert des Schirmwiderstands maximal 1 Megaohm betragen, während der Widerstandswert des Messwiderstands oberhalb von 100 Gigaohm beträgt. Der Schirmwiderstand ist dann niederohmig ausgebildet und bewirkt selbst vernachlässigbare Streukapazitäten.

Vorteilhafterweise sind die Abmessungen des Schirmwiderstands ähnlich denjenigen des Messwiderstands, um am Schirmwiderstand den gleichen Spannungsabfall wie am Messwiderstand zu bewirken. Der Messwiderstand ist typischerweise langgestreckt ausgebildet mit einer Länge, die größer ist als dessen Breite und Höhe. Vorzugsweise ist die Gesamtlänge des Schirmwiderstands größer als die Länge des Messwiderstands. Die über den Messwiderstand überstehenden Abschnitte des Schirmwiderstands weisen dann gleiche Potentiale auf.

Bei dem Schirmwiderstand kann es sich um einen Zylinder handeln, der den Messwiderstand umgibt und hierzu über den Messwiderstand geschoben ist. Diese Ausführungsform ist nicht Teil der Erfindung. Bei dem zylindrischen Schirmwiderstand kann es sich beispielsweise um ein Kohleröhrchen handeln, um neben den gewünschten geometrischen Abmessungen auch den gewünschten niederohmigen Widerstandswert zu erzielen.

Der Schirmwiderstand ist als Kette aus mehreren elektrischen Leitern gebildet, die den Messwiderstand jeweils zumindest teilweise umgeben. Zwischen benachbarten Leitern sind niederohmige Einzelwiderstände angeordnet. Ein erstes Ende der Kette weist dasselbe elektrische Potential auf wie das erste Ende des Messwiderstands. Das gegenüberliegende zweite Ende der Kette weist hingegen dasselbe Potential auf wie das gegenüberliegende zweite Ende des Messwiderstands. Die über den Messwiderstand überstehenden Teile des Schirmwiderstands weisen dieselben Potentiale auf wie die Enden des Messwiderstands. Die Einzelwiderstände weisen vorzugsweise jeweils einen Widerstandswert von maximal 100 Kiloohm auf.

Vorteilhafterweise weisen die elektrischen Leiter jeweils einen um den Messwiderstand herum gekrümmten oder abgewinkelten Abschnitt auf.

Im Folgenden werden anhand der Figuren Ausführungsbeispiele der Erfindung näher erläutert. Es zeigen:
- Fig. 1: ein schematisches Blockschaltbild,
- Fig. 2: ein erstes Ausführungsbeispiel in perspektivischer Ansicht,
- Fig. 3: die Ansicht gemäß dem Pfeil III in Fig. 2,
- Fig. 4: die Ansicht gemäß Fig. 3 eines zweiten Ausführungsbeispiels,
- Fig. 5: die Ansicht gemäß Fig. 3 eines dritten Ausführungsbeispiels, das nicht Teil der Erfindung ist. und
- Fig. 6: ein Schaltbild des ersten Ausführungsbeispiels.

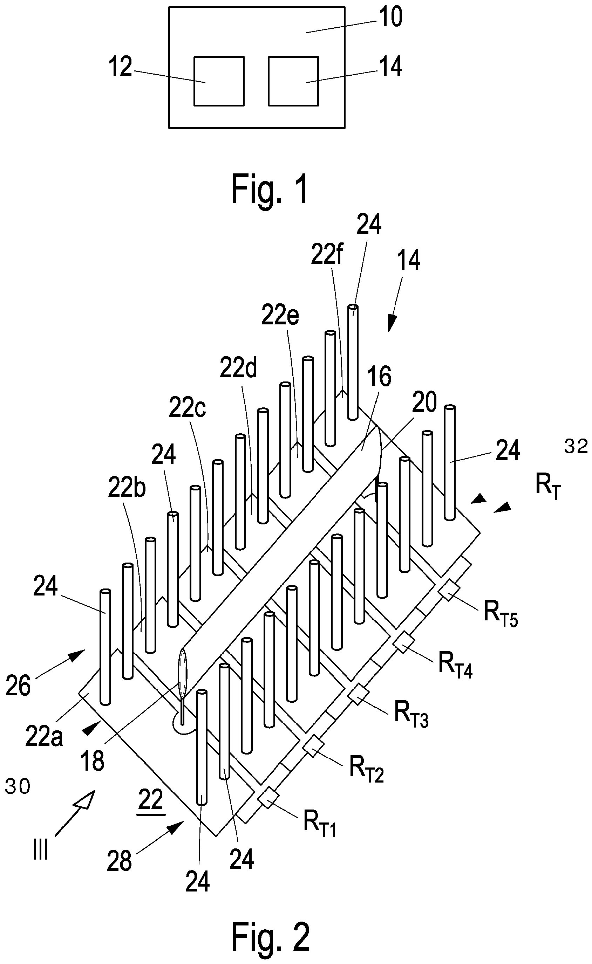

In Fig. 1 ist erkennbar, dass der Gasdetektor 10 eine Ionisiervorrichtung 12 und eine Messvorrichtung 14 aufweist. Bei der Ionisiervorrichtung kann es sich um ein Massenspektrometer handeln. Ein Detail der Messvorrichtung ist in Fig. 2 näher dargestellt.

Die Messvorrichtung 14 weist einen in Fig. 2 nicht dargestellten Operationsverstärker zur Verstärkung des Messstroms, mit einem Messwiderstand 16 auf. Der Messwiderstand 16 ist ein langgestrecktes Bauteil im vorliegenden Fall zylindrisch mit ovalem Querschnitt. Die Längenerstreckung des Messwiderstands ist größer als dessen Breite und Höhe. Ein erstes stirnseitiges Ende 18 und ein dem ersten Ende 18 gegenüberliegendes zweites stirnseitiges Ende 20 des Messwiderstands sind elektrisch mit dem Operationsverstärker verbunden und auf einer Fläche 22 mit elektrischen Leitern unterhalb des Messwiderstands 16 abgestützt.

Benachbart zu dem Messwiderstand 16 wird ein elektrischer Schirmwiderstand R_{T} aus mehreren elektrischen Leitern 22a bis 22f und 24 gebildet, wobei zwischen benachbarten Leitern 24 niederohmige Einzelwiderstände R_{T1}, R_{T2}, R_{T3}, R_{T4} und R_{T5} angeordnet und elektrisch mit den Leitern 24 verbunden sind. Die elektrischen Leiter 24 sind jeweils als vertikal aufragende Stiftpfosten ausgebildet und in zwei Reihen 26, 28 auf einander gegenüberliegenden Seiten des Messwiderstands 16 angeordnet. Die beiden Reihen 26, 28 sind gerade, parallel zueinander und parallel zu dem Messwiderstand 16 in jeweils gleichen Abständen zu dem Messwiderstand 16 angeordnet. Gegenüberliegende Leiter 24 sind elektrisch miteinander verbunden und bilden somit eine Kette aus mehreren elektrischen Leiterpaaren mit den Leitern 24. Das eine Ende 30 der Kette weist das selbe elektrische Potential auf wie das eine Ende 18 des Messwiderstands 16. Da der Messwiderstand am Ende 18 den Messstrom aufnehmen muss, ist dieser nicht elektrisch mit der Teilfläche 22a verbunden. Das andere, gegenüberliegende Ende 32 der Kette weist dasselbe Potential auf wie das andere Ende 20 des Messwiderstands 16.

Die elektrischen Leiterpaare mit den Leitern 24 sind als Doppelpaar angeordnet, wobei die Leiterpaare aus den Leitern 24 in Längsrichtung des Messwiderstands 16 jeweils zu einem Doppelpaar verbunden sind. Benachbarte Teilflächen 22a; 22b, 22c, 22d, 22e und 22f sind über einen der Einzelwiderstände R_{T1}-R_{T5} miteinander verbunden. Aufgrund des resultierenden Spannungsabfalls an dem jeweiligen Einzelwiderstand liegen die benachbarten Teilflächen und die zugehörigen elektrischen Leiter 24 benachbarter Doppelpaare auf verschiedenen elektrischen Potentialen.

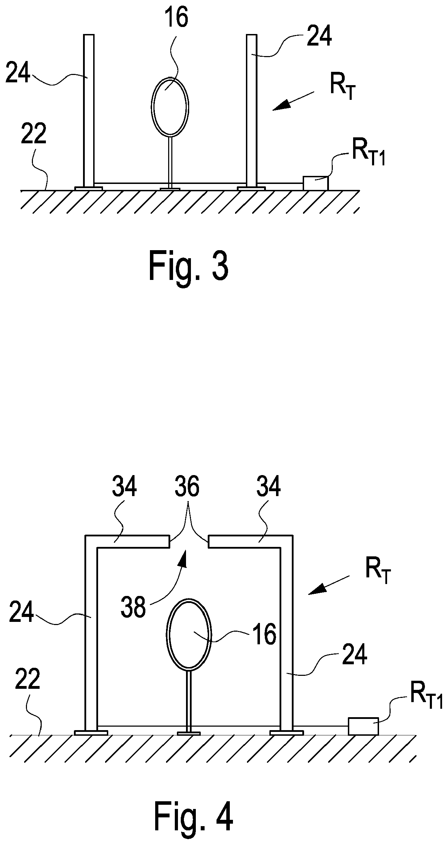

Die elektrischen Leiter 24 schirmen den Messwiderstand 16 auf lateral gegenüberliegenden Seiten ab. Unterhalb wird der Messwiderstand 16 durch die elektrisch leitenden Teilflächen 22a-22f abgeschirmt. Bei dem ersten Ausführungsbeispiel ist die der Fläche 22 gegenüberliegende Oberseite des Messwiderstands 16 nicht abgeschirmt, wie in Fig. 3 erkennbar.

Um die Oberseite des Messwiderstands 16 auch noch abzuschirmen, weisen die elektrischen Leiter 24 des in Fig. 4 dargestellten zweiten Ausführungsbeispiels jeweils einen abgewinkelten Abschnitt 34 auf. Jeder elektrische Leiter ist dadurch L-förmig ausgebildet. Die abgewinkelten Abschnitte 34 derselben Reihe 26 beziehungsweise 28 sind dabei parallel zueinander und gleich ausgerichtet. Die Enden 36 der elektrischen Leiter 24 verschiedener Reihen 26, 28 sind, wie in Fig. 4 erkennbar, aufeinander zugerichtet. Zwischen den Enden 36 der elektrischen Leiter verschiedener Reihen 26, 28 verbleibt ein geringer Spalt 38.

In Fig. 2 sind die Stiftpfosten der elektrischen Leiter zylindrisch ausgebildet. Alternativ sind andere Formen der elektrischen Leiter 24 denkbar, beispielsweise als flächige wandstückartige Elemente, deren Breite zum Beispiel der Breite einer elektrisch leitenden Teilfläche 22a-22f entspricht. Zwischen den benachbarten Teilflächen und den benachbarten Leitern 24 einer Reihe 26, 28 verbleibt in Längsrichtung vorzugsweise lediglich ein geringer Spalt zur elektrischen Isolierung, während andererseits durch die flächige Ausgestaltung der Teilflächen und der Leiter 24 eine große Abschirmung erreicht wird.

Die Leiter aus den Reihen 26 und 28 können dabei aus je einem zusammenhängenden gebogenen Leiter bestehen. Weiterhin können aus den beiden Reihen der Stiftpfosten jeweils zwei Stiftpfosten in Längsrichtung miteinander verbunden werden. Dabei wird die Hälfte der R_{T} Widerstände benötigt.

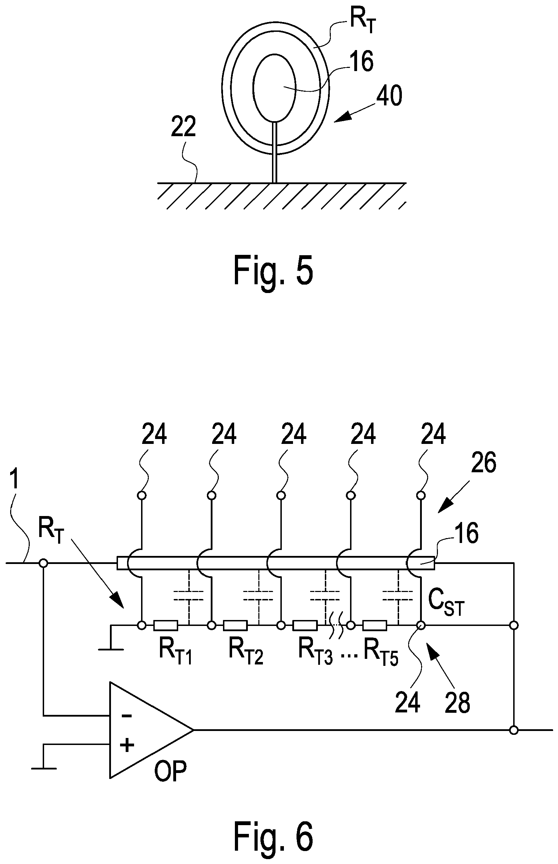

Bei dem in Fig. 5 dargestellten Ausführungsbeispiel, das nicht Teil der Erfindung ist, ist anstelle der elektrischen Leiter 24 ein als Zylinder 40 ausgebildeter Schirmwiderstand R_{T} vorgesehen, der den Messwiderstand 16 in Umfangsrichtung vollständig umgibt.

Fig. 6 zeigt ein elektrisches Schaltbild der Messvorrichtung 14 nach dem ersten Ausführungsbeispiel gemäß Fig. 2. Erkennbar ist, dass die Länge des Schirmwiderstands R_{T} der Gesamtlänge des Messwiderstands 16 entspricht beziehungsweise diese übertrifft Der Messwiderstand 16 und der Schirmwiderstand R_{T} sind Bestandteil des Rückkopplungspfades des Operationsverstärkers OP. Jeder der Einzelwiderstände R_{T1}-R_{T5} weist einen Widerstandswert im Bereich weniger Kiloohm auf.

In den Ausführungsbeispielen ist ein aus fünf Einzelwiderständen gebildeter Schirmwiderstand R_{T} dargestellt. Diese Anzahl von Einzelwiderständen ist lediglich beispielhaft. Bevorzugt sind zwischen jeden benachbarten elektrischen Teilflächen Einzelwiderstände angeordnet. Dabei ist denkbar, dass wie in Fig. 2 nur jede n-te Teilfläche über einen Einzelwiderstand mit der jeweils benachbarten Teilfläche verbunden ist, während die übrigen benachbarten Teilflächen ohne separaten Widerstand miteinander verbunden sind.

Das Ausführungsbeispiel in den Figuren 2 und 6 zeigt einen Schirmwiderstand R_{T}, dessen Gesamtlänge größer ist als die Länge des Messwiderstands 16. Alternativ ist denkbar, dass die Gesamtlänge des Schirmwiderstands R_{T} gleich groß wie die Länge des Messwiderstands 16 ist. Hierzu können weitere Teilflächen mit zugehörigen Paaren weiterer elektrischer Leiter 24 in den über die beiden stirnseitigen Enden 18, 20 des Messwiderstands 16 hinausragenden Bereichen angeordnet sein. Diese zusätzlichen Teilflächen sollten durch eine elektrisch leitende Verbindung auf demselben Potential liegen.

Zudem ist eine in den Figuren nicht dargestellte elektrische Abschirmung jedes stirnseitigen Endes 18, 20 des Messwiderstands 16 denkbar, beispielsweise in Form einer elektrisch leitenden vertikal aufragenden Wand im Bereich jedes stirnseitigen Endes 18, 20. Jede dieser aufragenden Wände sollte dann mit einer zugehörigen elektrischen Teilfläche verbunden sein bzw. auf demselben Potential wie diese liegen.

## Patentansprüche

1. Gasdetektor (10) mit
einer Ionisiervorrichtung (12) zur Erzeugung von Ionen in Abhängigkeit von einem zu detektierenden Gas, mit einem Fänger zur Aufnahme des elektrischen Stroms, der durch die Ionen erzeugt wird, und
einer Messvorrichtung (14) mit einem elektrischen Messwiderstand (16), der aus dem Strom ein elektrisches Messpotential erzeugt,
wobei der Messwiderstand zumindest teilweise von einem elektrischen Schirmwiderstand R_{T} umgeben ist und
wobei an einander gegenüberliegenden Bereichen des Messwiderstands und des Schirmwiderstands R_{T} bis auf eine Abweichung von höchstens 25 % dasselbe Potenzial anliegt,
**dadurch gekennzeichnet,**
**dass** der Schirmwiderstand R_{T} als Kette aus mehreren elektrischen Leitern (24), die den Messwiderstand jeweils zumindest teilweise umgeben, gebildet ist, wobei zwischen benachbarten Leitern (24) Einzelwiderstände (R_{T1...}R_{TN}) angeordnet sind, wobei das eine Ende (30) der Kette dasselbe Potential wie das eine Ende (18) des Messwiderstands (16) aufweist und das andere Ende (32) der Kette dasselbe Potential wie das andere Ende (20) des Messwiderstands aufweist,
**dass** jeweils zwei elektrische Leiter (24) auf einander gegenüberliegenden Seiten des Messwiderstands (16) paarweise auf einer elektrisch leitenden Teilfläche (22a-22f) einer Fläche (22) unterhalb des Messwiderstands angeordnet und elektrisch mit dieser verbunden sind,
**dass** die elektrischen Leiter (24) jeweils als Stiftpfosten ausgebildet sind, und
**dass** die elektrischen Leiter (24) auf einander gegenüberliegenden Seiten des Messwiderstands (16) aufragen.

2. Gasdetektor (10) nach Anspruch 1, **dadurch gekennzeichnet, dass** eines der beiden Enden des Messwiderstands mit einem der beiden Enden des Schirmwiderstands R_{T} elektrisch verbunden ist, sodass diese Enden dasselbe Potenzial aufweisen.

3. Gasdetektor (10) nach Anspruch 2, **dadurch gekennzeichnet, dass** die jeweils anderen Enden des Messwiderstands und des elektrischen Schirmwiderstands R_{T} bis auf die Abweichung von höchstens 25 % auf Massepotenzial liegen.

4. Gasdetektor (10) nach einem der Ansprüche 1-3, **dadurch gekennzeichnet, dass** der Schirmwiderstand R_{T} einen geringeren Widerstandswert als der Messwiderstand (16) hat.

5. Gasdetektor (10) nach Anspruch 1 oder 2, **dadurch gekennzeichnet, dass** der Widerstandswert des Schirmwiderstands R_{T} maximal ein Megaohm beträgt und der Widerstandswert des Messwiderstands (16) größer als 1 Gigaohm ist.

6. Gasdetektor (10) nach einem der Ansprüche 1-5, **dadurch gekennzeichnet, dass** der Messwiderstand (16) langgestreckt ausgebildet ist mit einer Länge, die größer ist als dessen Höhe und Breite, wobei sich der Schirmwiderstand R_{T} über mindestens einen überwiegenden Teil der Länge des Messwiderstands (16) erstreckt.

7. Gasdetektor (10) nach einem der Ansprüche 1-6, **dadurch gekennzeichnet, dass** die Länge des Schirmwiderstands R_{T} größer als die Länge des Messwiderstands (16) ist, wobei der Teil des Schirmwiderstands, der jeweils unterschiedliche Potentiale aufweist so lang wie der aktive Bereich des Messwiderstands (16) ist.

8. Gasdetektor (10) nach einem der Ansprüche 1-7, **dadurch gekennzeichnet, dass** die elektrischen Leiter (24) einen um den Messwiderstand (16) herum gekrümmten oder abgewinkelten Abschnitt (34) aufweisen.

9. Gasdetektor (10) nach einem der vorherigen Ansprüche, **dadurch gekennzeichnet, dass** jeweils zwei benachbarte Leiter (24) zu einem Doppelpaar elektrisch zusammengeschaltet sind.

## Claims

1. A gas detector (10) comprising
an ionizing device (12) for producing ions depending on a gas to be detected, with a catcher for receiving the electrical current produced by the ions, and
a measuring device (14) with an electrical measuring resistor (16) which produces an electrical measuring potential from the current,
wherein the measuring resistor is surrounded at least in part by an electrical shield resistor R_{T}, and
the same potential is applied to mutually opposed regions of the measuring resistor and the shield resistor R_{T} up to a deviation of at most 25%,
**characterized in that**
the shield resistor R_{T} is formed as a chain of a plurality of electrical conductors (24), each of which surround at least in part the measuring resistor, wherein individual resistors (R_{T1...}R_{TN}) are arranged between adjacent conductors (24), wherein the one end (30) of the chain has the same potential as the one end (18) of the measuring resistor (16) and the other end (32) of the chain has the same potential as the other end (20) of the measuring resistor,
in each case two electrical conductors (24) are arranged in pairs on mutually opposing sides of the measuring resistor (16) on an electrically conductive partial surface (22a-22f) of a surface (22) below the measuring resistor and are electrically connected thereto,
the electrical conductors (24) are each designed as pin posts, and
the electrical conductors (24) protrude on mutually opposed sides of the measuring resistor (16).

2. The gas detector (10) according to claim 1, **characterized in that** one of the two ends of the measuring resistor is electrically connected to one of the two ends of the shield resistor R_{T}, so that said ends have the same potential.

3. The gas detector (10) according to claim 2, **characterized in that** the respective other ends of the measuring resistor and the electrical shield resistor R_{T} are at ground potential up to a deviation of at most 25%.

4. The gas detector (10) according to any one of claims 1-3, **characterized in that** the shield resistor R_{T} has a lower resistance value than the measuring resistor (16).

5. The gas detector (10) according to claim 1 or 2, **characterized in that** the resistance value of the shield resistor R_{T} is a maximum of one megohm and the resistance value of the measuring resistor (16) is greater than 1 gigaohm.

6. The gas detector (10), according to any one of claims 1-5, **characterized in that** the measuring resistor (16) is elongated in form with a length which is greater than the height and width thereof, wherein the shield resistor R_{T} extends over at least a predominant part of the length of the measuring resistor (16).

7. The gas detector (10), according to any one of claims 1-6, **characterized in that** the length of the shield resistor R_{T} is greater than the length of the measuring resistor (16), wherein the part of the shield resistor which has different potentials in each case is as long as the active region of the measuring resistor (16).

8. The gas detector (10) according to any one of claims 1-7, **characterized in that** the electrical conductors (24) have a section (34) which is curved or angled around the measuring resistor (16).

9. The gas detector (10) according to any one of the preceding claims, **characterized in that** two adjacent conductors (24) are each electrically connected together to form a double pair.

## Revendications

1. Détecteur de gaz (10) comprenant
un dispositif ionisant (12) pour produire des ions en fonction d'un gaz à détecter, comprenant un piège pour recevoir le courant électrique qui est produit par les ions, et
un dispositif de mesure (14) comprenant une résistance de mesure électrique (16) qui produit un potentiel de mesure électrique à partir du courant,
la résistance de mesure étant entourée au moins partiellement d'une résistance de blindage électrique R_{T} et
le même potentiel s'appliquant à des zones opposées de la résistance de mesure et de la résistance de blindage R_{T}, jusqu'à un écart maximum de 25 % ;
**caractérisé**
**en ce que** la résistance de blindage R_{T} est formée sous la forme d'une chaîne constituée de plusieurs conducteurs électriques (24) qui entourent respectivement au moins partiellement la résistance de mesure, des résistances individuelles (R_{T1}...R_{TN}) état agencées entre des conducteurs adjacents (24), la première extrémité (30) de la chaîne présentant le même potentiel que la première extrémité (18) de la résistance de mesure (16) et l'autre extrémité (32) de la chaîne présentant le même potentiel que l'autre extrémité (20) de la résistance de mesure,
**en ce que** deux conducteurs électriques (24) sont respectivement agencés en paire sur des côtés opposés de la résistance de mesure (16) sur une surface partielle (22a-22f) électriquement conductrice d'une surface (22) en dessous de la résistance de mesure et sont reliés électriquement à celle-ci,
**en ce que** les conducteurs électriques (24) sont respectivement formés sous la forme de montants de broche, et
**en ce que** les conducteurs électriques (24) font saillie sur des côtés opposés de la résistance de mesure (16).

2. Détecteur de gaz (10) selon la revendication 1, **caractérisé en ce que** l'une des deux extrémités de la résistance de mesure est connectée électriquement à l'une des deux extrémités de la résistance de blindage R_{T} de sorte que ces extrémités présentent le même potentiel.

3. Détecteur de gaz (10) selon la revendication 2, **caractérisé en ce que** les autres extrémités respectivement de la résistance de mesure et de la résistance de blindage électrique R_{T} se situent au potentiel de masse jusqu'à un écart maximum de 25 %.

4. Détecteur de gaz (10) selon l'une quelconque des revendications 1 à 3, **caractérisé en ce que** la résistance de blindage R_{T} a une valeur de résistance inférieure à la résistance de mesure (16).

5. Détecteur de gaz (10) selon la revendication 1 ou 2, **caractérisé en ce que** la valeur de résistance de la résistance de blindage R_{T} est au maximum un mégaohm et la valeur de résistance de la résistance de mesure (16) est supérieure à 1 gigaohm.

6. Détecteur de gaz (10) selon l'une quelconque des revendications 1 à 5, **caractérisé en ce que** la résistance de mesure (16) est formée s'étendant longitudinalement avec une longueur qui est supérieure à sa hauteur et à sa largeur, la résistance de blindage R_{T} s'étendant sur au moins une majeure partie de la longueur de la résistance de mesure (16).

7. Détecteur de gaz (10) selon l'une quelconque des revendications 1 à 6, **caractérisé en ce que** la longueur de la résistance de blindage R_{T} est supérieure à la longueur de la résistance de mesure (16), la partie de la résistance de blindage qui présente respectivement des potentiels différents étant aussi longue que la zone active de la résistance de mesure (16).

8. Détecteur de gaz (10) selon l'une quelconque des revendications 1 à 7, **caractérisé en ce que** les conducteurs électriques (24) présentent une section (34) incurvée ou coudée autour de la résistance de mesure (16).

9. Détecteur de gaz (10) selon l'une quelconque des revendications précédentes, **caractérisé en ce que** respectivement deux conducteurs adjacents (24) sont interconnectés électriquement en double paire.
